# FASCICULE DE BREVET EUROPEEN

(11) **EP 2 916 805 B1**
(45) Date de publication et mention de la délivrance du brevet: **18.07.2018**
(21) Numéro de dépôt: 13801637.3
(22) Date de dépôt: 07.11.2013
(51) Int. Cl.: A61K 8/365, A61Q 5/04, A61K 8/42, A61K 8/73, A61K 8/44

(54) **COMPOSITION COMPRENANT UN COMPOSE DICARBONYLE ET PROCEDE DE LISSAGE DES CHEVEUX A PARTIR DE CETTE COMPOSITION**
ZUSAMMENSETZUNG MIT EINER DICARBONYLVERBINDUNG UND HAARGLÄTTUNGSVERFAHREN UNTER VERWENDUNG DIESER ZUSAMMENSETZUNG
COMPOSITION COMPRISING A DICARBONYL COMPOUND AND METHOD FOR SMOOTHING THE HAIR USING THIS COMPOSITION

(30) Priorité: 09.11.2012 FR 1260658
(43) Date de publication de la demande: 16.09.2015
(73) Titulaire: L'Oréal, 75008 Paris (FR)
(72) Inventeur: BIATO, Camila, CEP : 22795-295 Rio de Janeiro (BR); SILVESTRE, Liliane, CEP:21235-602 Rio de Janeiro - RJ (BR)
(74) Mandataire: Leray, Noelle
(86) Numéro de dépôt international: PCT/FR2013/052668
(87) Numéro de publication internationale: WO 2014/072644

(56) Documents cités:
- WO-A1-93/00882
- WO-A1-2007/135299
- WO-A1-2012/105985
- WO-A2-2011/104282
- WO-A2-2012/010351
- DE-C1- 19 504 914
- GB-A- 2 002 833
- JP-A- S5 692 810
- JP-A- 2004 002 459

## Description

La présente invention concerne une composition cosmétique, notamment capillaire, à base de composés dicarbonylés particuliers ainsi qu'un procédé de lissage des fibres kératiniques tels que les cheveux à partir de cette composition.

Dans le domaine capillaire, les consommateurs souhaitent disposer de compositions permettant d'apporter un changement temporaire à leur chevelure, et ce en visant une bonne tenue de l'effet réalisé. En général, on désire que le changement persiste aux shampooings pendant au minimum 15 jours, voire plus selon la nature dudit changement.

Des traitements existent déjà pour modifier la couleur ou la forme des cheveux ainsi que, dans une certaine mesure, la texture des cheveux. L'un des traitements connus pour modifier la texture des cheveux consiste en l'association de chaleur et d'une composition comprenant du formol. Ce traitement est spécialement efficace pour conférer un meilleur aspect aux cheveux abîmés, et/ou pour traiter les cheveux longs et les cheveux bouclés.

On associe l'action du formol à sa capacité de réticuler les protéines par réaction sur ses sites nucléophiles. La chaleur utilisée peut être celle du fer (pince plate ou fer à friser), dont la température peut atteindre en général 200°C ou plus. Toutefois, on cherche de plus en plus à éviter l'emploi de telles substances, qui peuvent se révéler agressives pour le cheveu et les autres matières kératiniques.

Il a ainsi été proposé, par la demande WO2011/104282, un nouveau procédé pour lisser de manière semi-permanente les cheveux, consistant à appliquer une solution d'alphacétoacide sur le cheveu pendant 15 à 120 minutes, puis à sécher et enfin à lisser au fer, à une température d'environ 200°C, la chevelure. L'alphacétoacide employé est de préférence l'acide glyoxylique.

Les documents WO2012/010351 et WO2012/105985 décrivent également des procédés de lissage utilisant l'acide glyoxylique.

Toutefois, on a constaté que l'utilisation de l'acide glyoxylique pouvait engendrer quelques limitations importantes; en particulier, quand le cuir chevelu est sensible et/ou irrité. Sa volatilité, amplifiée par l'utilisation de chaleur (fer), peut également poser problème.

Il est aussi connu du document WO2007/135299 d'utiliser des dérivés d'alpha hydroxy-acides et/ou de céto-acides dans des procédés de défrisage.

Le document GB2002833 décrit également l'utilisation de glyoxal dans un procédé de défrisage.

Il est déjà connu d'utiliser des esters d'acide glyoxylique dans des compositions capillaires, en particulier dans des compositions de coloration des cheveux tel que décrit dans le document DE19859722 et dans des compositions réductrices tel que décrit dans le document DE19860239.

L'efficacité de ces compositions n'est cependant pas suffisante.

Le but de l'invention est de développer une composition de lissage/défrisage stable dans le temps et qui permet de lisser/défriser et/ou de réduire le volume les fibres kératiniques, en particulier cheveux, de façon efficace et rémanente en limitant la dégradation des cheveux tout en conservant un confort au moment de l'application pour l'utilisateur de la composition mais aussi pour le coiffeur qui l'applique.

Ainsi, un objet de la présente invention est une composition comprenant
- au moins un composé dicarbonylé répondant à la formule (I) suivante
   - 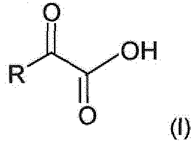 formule (I) dans laquelle
      R représente un atome ou groupe choisi parmi i) hydrogène, ii) carboxy -C(O)OH, iii) alkyle linéaire ou ramifié en C₁-C₆ éventuellement substitué de préférence par au moins un radical hydroxy -OH, carboxy ou halogène tel que Br ; iv) phényle éventuellement substitué, v) benzyle éventuellement substitué, iv) et v) étant de préférence éventuellement substitués par au moins un radical -OH ou -C(O)OH ; vi) un radical indolyle et vii) un radical imidazolylméthyle et ses tautomères tel que 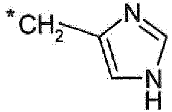 avec * représentant la partie reliée au reste de la molécule,
      et/ou ses dérivés choisis parmi les esters du ou des groupes carboxy, les amides du ou des groupes carboxy, les (thio)acétals et hémi(thio)acétals de la ou des fonctions carbonyles des composés de formule (I), sous forme libre ou éventuellement sous forme de sels ou d'hydrates, et/ou ses hydrates et/ou les sels correspondants ;
- au moins un polymère cellulosique, et
- au moins un tensioactif amphotère ou zwittérionique, la composition présentant un pH inférieur à 4.

L'invention a aussi pour objet un procédé de lissage des fibres kératiniques, en particulier des cheveux, qui comprend l'application sur lesdites fibres de la composition de l'invention suivi d'une étape de lissage au moyen d'un fer de lissage à une température d'au moins 150°C, de préférence entre 150 et 250°C. La composition de l'invention est stable. La composition de l'invention, et le procédé de traitement des fibres kératiniques mettant en oeuvre les composes de formule (I), les polymères cellulosiques et des tensioactifs amphotères tels que définis précédemment, permettent un bon lissage des fibres kératiniques en limitant la dégradation de ces fibres kératiniques, même lorsque l'application de la ou des compositions est suivie par un traitement thermique, notamment au moyen de fer de lissage des cheveux et présentent une qualité d'usage apprécié, notamment sans vaporisation excessive de la composition au moment du lissage. La composition et le procédé de traitement des fibres kératiniques selon l'invention permettent aussi de limiter le changement de la couleur des fibres, ainsi que les problèmes de casse desdites fibres telles que les cheveux. La composition et le procédé de l'invention vont en outre améliorer les propriétés physiques des cheveux, en réduisant l'effet de frisottis de façon durable.

Dans ce qui suit l'expression « au moins un » est équivalente à l'expression « un ou plusieurs ».

De préférence, la composition selon l'invention ne comprend ni agent colorant ni agent réducteur.

Par « agents colorants », on entend selon la présente invention des agents de coloration des fibres kératiniques tels que les colorants directs, les pigments ou les précurseurs de colorant d'oxydation (bases et coupleurs). S'ils sont présents, leur teneur ne dépasse pas 0,001 % en poids par rapport au poids total de la composition. En effet, à une telle teneur, seule la composition serait teintée, c'est-à-dire qu'on n'observerait pas d'effet de coloration des fibres kératiniques.

On rappelle que les précurseurs de colorants d'oxydation, bases d'oxydation et coupleurs, sont des composés peu ou non colorés qui par une réaction de condensation en présence d'un agent oxydant, donnent une espèce colorée. Quant aux colorants directs, ces composés sont colorés et présentent une certaine affinité pour les fibres kératiniques.

Par « agent réducteur », on entend selon la présente invention un agent capable de réduire les liaisons disulfures des cheveux, tel que les composés choisis parmi les thiols, les sulfites alcalins, les hydrures, les phosphines.

Dans la présente invention, les composés dicarbonylés de formule (I) ou leurs dérivés peuvent se présenter sous forme libre mais aussi sous leurs formes hydrates ou sous forme de leurs sels, de préférence sous forme libre ou d'hydrates.
En tant que « *dérivés* » des composés dicarbonylés de formule (I), on peut citer les esters du ou des groupes carboxy, les amides du ou des groupes carboxy, les (thio)acétals et hémi(thio)acétals de la ou des fonctions carbonyles des composés de formule **(I),** sous forme libre ou éventuellement sous forme de sels ou d'hydrates, de préférence sous forme libre ou d'hydrates.

Les esters et amides peuvent être synthétisés à partir des procédés d'estérification ou d'amidification classiques à partir des acides correspondants bien connus par l'homme du métier.

Les esters sont par exemple obtenus à partir des acides de formule (I) et d'un mono ou polyalcool.

Par « *mono ou poly alcool* », on entend un composé organique comprenant un groupe hydroxy (monoalcool) ou au moins deux groupes hydroxy (polyalcool ou polyol) ; ledit composé organique hydroxylé pouvant être aliphatique, acyclique, linéaire ou ramifié, ou (hétéro)cyclique, tels que les sucres (mono ou polysaccharides) ou les sucres alcools

Plus particulièrement, le polyalcool comprend de 2 à 100 groupes hydroxy ; et préférentiellement de 2 à 20 groupes hydroxy ; encore plus préférentiellement de 2 à 10 groupes hydroxy, mieux 2 ou 3 groupes hydroxy .

De préférence le mono ou polyalcool est choisi parmi le méthanol, l'éthanol, le propanol, l'isopropanol, le butanol, l'hexanol, l'éthylène gllycol, le glycérol, la dihydroxyacétone, le glucose, le sorbitol, le menthol.

Les amides sont par exemple obtenus à partir de l'acide de formule (I) et d'une mono ou polyamine organique.

Par « mono ou polyamine », on entend un composé organique comprenant un groupe amino(monoamine) ou au moins deux (et de préférence de 2 à 100, mieux de 2 à 20) groupes amino ; le dit composé organique pouvant être aliphatique, acyclique, linéaire ou ramifié ou (hétéro)cyclique.

Par groupe « amino » on entend un groupe amine primaire -NH₂, ou secondaire >NH.

De préférence la mono ou polyamine est aliphatique.

Cette amine est de préférence choisie parmi la méthylamine, l'éthylamine, la propylamine, l'isopropylamine, la butylamine, l'hexylamine, la monoéthanolamine, la monopropanolamine, la propane-1,2,3-triamine et la diaminoacétone.

Les acétals et hémiacétals des acides de formule (I) peuvent par exemple être obtenus à partir de la réaction d'alcools sur des formes bloquées des acides puis hydrolyse .Les alcools peuvent être les mêmes que ceux cités pour les esters. Les acétals peuvent également être des acétals cycliques.

Les sels peuvent être des sels issus de l'interaction des composés de formule (I) avec des acides ou des bases, les acides ou bases pouvant être de nature organique ou minérale.

De préférence les sels sont des sels issus de l'interaction des composés de formule (I) avec des bases. On citera en particuliers les sels de métaux alcalins alcalins ou alcalino tereux et en particulier les sels de sodium.

Dans la suite de la description, on nommera toutes ces variantes de composés dicarbonylés de formule (I) « composés dicarbonylés et/ou ses dérivés »

Selon un mode de réalisation particulier de l'invention le ou les composés dicarbonylés sont de formule **(I)** avec R représentant i) un atome d'hydrogène ou ii) un groupe alkyle linéaire ou ramifié en C₁-C₆ éventuellement substitué par un groupe carboxy.

Selon un mode de réalisation plus particulier, les composés de formule (I) sont choisis parmi l'acide glyoxylique, l'acide pyruvique, l'un de leurs dérivés, de leurs sels et leurs hydrates.

Préférentiellement le ou les composés dicarbonylés de formule (I) de l'invention sont choisis parmi l'acide glyoxylique et ses dérivés: acide glyoxylique ainsi que sa forme hydrate (HO)₂CH-C(O)-OH. Par exemple l'acide glyoxylique en solution aqueuse à 50 % vendu par la société MERCK.

En tant que dérivés d'acide glyoxylique, on peut citer les esters d'acide glyoxylique, les amides d'acide glyoxylique, les (thio)acétals et hémi(thio)acétals d'acide glyoxylique, les (thio)acétals et hém(thio)iacétals d'ester d'acide glyoxylique.

Les esters d'acide glyoxylique sont par exemple obtenus à partir d'acide glyoxylique et d'un mono ou polyalcool, notamment ceux cités précédemment.

De préférence le mono ou polyalcool est choisi parmi le méthanol, l'éthanol, le propanol, l'isopropanol, le butanol, l'hexanol, l'éthylène gllycol, le glycérol, la dihydroxyacétone, le glucose, le sorbitol, le menthol.

On peut en particulier citer à titre d'esters le glyoxylate de méthyle, le glyoxylate d'éthyle, le glyoxylate de glycérol, le glyoxylate de dihydroxyacétone, le diglyoxylate ou triglyoxylate de de glycérol, les mono,di ou tri glyoxylate de sorbitol, les mono,di ou tri glyoxylate de glucose, le glyoxylate de menthyle ,leurs acétals, hémiacétals , hydrates.

Les amides d'acide glyoxylique sont par exemple obtenus à partir d'acide glyoxylique et d'une mono ou polyamine organique, notamment celles décrites précédemment.

Cette amine est de préférence choisie parmi la méthylamine, l'éthylamine,la propylamine, l'isopropylamine,la butylamine, l'hexylamine, la monoéthanolamine, la monopropanolamine, la propane-1,2,3-triamine et la diaminoacétone.

On peut en particulier citer le N-bétahydroxyéthylamide de l'acide glyoxylique et le N-gammahydroxypropylamide de l'acide glyoxylique leurs acétals, hémiacétals , hydrates

Les (thio)acétals et hémi(thio)acétals d'acide glyoxylique peuvent par exemple être obtenus à partir de la réaction d'alcools pour les acétals ou hémiacétals ou de thiols pour les thioacétals ou hémithioacétals sur des formes bloquées d'acide glyoxylique puis hydrolyse. Les alcools peuvent être les mêmes que ceux cités pour les esters. Les thiols peuvent être des équivalents (appelés mono ou polythiols) aux mono ou polyalcools cités supra à ceci près que la ou les fonctions hydroxy desdits mono ou polyalcools sont remplacées par une ou des fonctions thiols SH des mono ou polythiols. Les acétals ou thioacétals peuvent également être des (thio)acétals cycliques.

On peut en particulier citer l'acide diméthoxy acétique, l'acide diéthoxy acétique, l'acide 1,3-dioxane 2 carboxylique, l'acide 1,3-dioxolane 2-carboxylique.

Selon un mode de réalisation particulièrement préféré, le composé de formule (I) est l'acide glyoxylique sous forme hydrate.

Ainsi, le procédé de la présente invention est mis en oeuvre sans une étape de déformation permanente à pH basique ni à base de réducteur. Les compositions selon l'invention peuvent se présenter sous toutes les formes galéniques classiquement utilisées, et notamment sous forme d'une solution ou suspension aqueuse, alcoolique ou hydroalcoolique, ou huileuse; d'une solution ou d'une dispersion du type lotion ou sérum; d'une émulsion, notamment de consistance liquide ou semi-liquide, du type H/E, E/H ou multiple; d'une suspension ou émulsion de consistance molle de type crème (H/E) ou (E/H); d'un gel aqueux ou anhydre, ou de toute autre forme cosmétique.

Ces compositions peuvent être conditionnées dans des flacons pompes ou dans des récipients aérosols, afin d'assurer une application de la composition sous forme vaporisée (laque) ou sous forme de mousse. De telles formes de conditionnement sont indiquées, par exemple, lorsqu'on souhaite obtenir un spray ou une mousse, pour le traitement des cheveux. Dans ces cas, la composition comprend de préférence au moins un agent propulseur.

Les compositions de l'invention peuvent être aqueuses ou anhydres. Elles sont de préférence aqueuses et comprennent alors de l'eau à une concentration allant de 5 à 98%, mieux de 5 à 50%, encore mieux de 10 à 40 % en poids par rapport au poids total de la composition.

Selon un mode de réalisation la composition de l'invention se trouve sous forme d'une composition aqueuse comprenant de 0,1 à 20% de composés dicarbonylés de formule (I) et/ou de ses dérivés, de préférence au moins 3 % de composés dicarbonylés de formule (I) et/ou de ses dérivés, préférentiellement de 3 à 10 % en poids du poids total de la composition.

Selon l'invention, la composition comprend au moins un polymère cellulosique. Les polymères cellulosiques peuvent être des polymères anioniques, cationiques, amphotères ou nonioniques.

Par polymère « *cellulosique* », on entend selon l'invention tout composé polysaccharidique possédant dans sa structure des enchaînements de résidus glucose unis par des liaisons β-1,4; outre les celluloses non substituées, les dérivés de celluloses peuvent être anioniques, cationiques, amphotères ou nonioniques. Ainsi, les polymères cellulosiques de l'invention peuvent être choisis parmi les celluloses non substituées y compris sous une forme microcristalline et les éthers de cellulose. Parmi ces polymères cellulosiques, on distingue les éthers de celluloses, les esters de celluloses et les esters éthers de celluloses. Parmi les esters de celluloses, on trouve les esters inorganiques de cellulose (nitrates, sulfates ou phosphates de cellulose...), les esters organiques de cellulose (monoacétates, triacétates, amidopropionates, acétatebutyrates, acétatepropionates ou acétatetrimellitates de cellulose....) et les esters mixtes organique/inorganique de cellulose tels que les acétatebutyratesulfates et les acétatepropionatesulfates de cellulose. Parmi les esters éthers de cellulose, on peut citer les phtalates d'hydroxypropylméthylcellulose et les sulftates d'éthylcellulose.

Selon une première variante, le ou les polymères cellulosiques sont non ioniques. A titre d'exemple, on peut citer les (C₁-C₄)alkylcelluloses telles que les méthylcelluloses et les éthylcelluloses (par exemple Ethocel standard 100 Premium de DOW CHEMICAL) ; les (poly)hydroxy(C₁-C₄)alkylcelluloses telles que les hydroxyméthylcelluloses, les hydroxyéthylcelluloses (par exemple Natrosol 250 HHR proposé par AQUALON) et les hydroxypropylcelluloses (par exemple Klucel EF d'AQUALON); les celluloses mixtes (poly)hydroxy(C₁-C₄)alkyl-(C₁-C₄)alkylcelluloses telles que les hydroxypropyl-méthylcelluloses (par exemple Methocel E4M de DOW CHEMICAL), les hydroxyéthyl-méthylcelluloses, les hydroxyéthyl-éthylcelluloses (par exemple Bermocoll E 481 FQ d'AKZO NOBEL) et les hydroxybutyl-méthylcelluloses.

Selon une deuxième variante, le ou les polymères cellulosiques sont anioniques. Parmi les éthers de cellulose anioniques, on peut citer les (poly)carboxy(C₁-C₄)alkylcelluloses et leurs sels. A titre d'exemple, on peut citer les carboxyméthylcelluloses, les carboxyméthylméthylcelluloses (par exemple Blanose 7M de la société AQUALON) et les carboxyméthylhydroxyéthylcelluloses et leurs sels de sodium.

Selon une troisième variante, le ou les polymères cellulosiques sont cationiques. Parmi les éthers de cellulose cationiques on peut citer les dérivés de cellulose cationiques tels que les copolymères de cellulose ou les dérivés de cellulose greffés avec un monomère hydrosoluble d'ammonium quaternaire, et décrits notamment dans le brevet US 4 131 576, tels que les (poly)hydroxy(C₁-C₄)alkyl celluloses, comme les hydroxyméthyl-, hydroxyéthyl- ou hydroxypropyl celluloses greffées notamment avec un sel de méthacryloyléthyl triméthylammonium, de méthacrylmidopropyl triméthylammonium, de diméthyl-diallylammonium. Les produits commercialisés répondant à cette définition sont plus particulièrement les produits vendus sous la dénomination "Celquat® L 200" et "Celquat® H 100" par la Société National Starch.

Selon un mode de réalisation particulier, les polymères cellulosiques de l'invention sont des polymères cellulosiques non ioniques et comprenant des chaines alkyles comportant de 1 à 6 atomes de carbone. De préférence, le ou les polymères cellulosiques de l'invention sont choisis parmi les éthers de celluloses en particulier les celluloses hydroxyalkyl-alkyl celluloses telles que les (poly)hydroxy(C₁-C₄)alkyl-(C₁-C₄)alkylcelluloses en particulier les hydroxypropyl-méthylcelluloses (par exemple Methocel F4M de DOW CHEMICAL), les hydroxyéthyl-méthylcelluloses, les hydroxyéthyl-éthylcelluloses (par exemple Bermocoll E 481 FQ d'AKZO NOBEL) et les hydroxybutyl-méthylcelluloses.

Les polymères cellulosiques selon la présente l'invention sont des polymères cellulosiques qui de préférence ne comportent pas de chaîne grasse i.e. qui de préférence ne comportent pas de chaine comprenant plus de 10 atomes de carbone.

Le ou les polymères cellulosiques de l'invention peuvent être présents dans la composition tinctoriale de l'invention dans des teneurs allant de 0,05 % à 10 % en poids, en particulier de 0,1 % à 5 % en poids, et mieux encore de 0,1 % à 3 % en poids, par rapport au poids total de la composition.

Selon un mode de réalisation particulier, la composition comprend au moins un tensio-actif amphotère ou zwittérionique.

En particulier, le ou les tensioactifs amphotères ou zwittérioniques, de préférence non siliconés, utilisables dans la présente invention, peuvent être notamment des dérivés d'amines aliphatiques secondaire ou tertiaire, éventuellement quaternisées, dans lesquels le groupe aliphatique est une chaîne linéaire ou ramifiée comportant de 8 à 22 atomes de carbone, lesdits dérivés d'amines contenant au moins un groupe anionique tel que, par exemple, un groupe carboxylate, sulfonate, sulfate, phosphate ou phosphonate.

On peut citer en particulier les alkyl(C₈-C₂₀)bétaïnes, les alkyl(C₈-C₂₀) sulfobétaïnes, les alkyl(C₈-C₂₀)amidoalkyl(C₃-C₈)bétaïnes et les alkyl(C₈-C₂₀)-amidalkyl(C₆-C₈)sulfobétaïnes.

Parmi les dérivés d'amines aliphatiques secondaires ou tertiaires, éventuellement quaternisées utilisables, tels que définis ci-dessus, on peut également citer les composés de structures respectives **(B1)** et **(B2)** suivantes :

Rₐ-C(O)-NH-CH₂-CH₂-N⁺(R_{b})(R_{c})-CH₂C(O)O⁻, M⁺, X⁻ **(B1)**

Formule dans laquelle :
▪ Rₐ représente un groupe alkyle ou alcényle en C₁₀-C₃₀ dérivé d'un acide RₐCOOH, de préférence présent dans l'huile de coprah hydrolysée, un groupe heptyle, nonyle ou undécyle ;
▪ R_{b} représente un groupe bêta-hydroxyéthyle ; et
▪ R_{c} représente un groupe carboxyméthyle ;
▪ M⁺ représente un contre ion cationique issu d'un métal alcalin, alcalinoterreux, tel que le sodium, un ion ammonium ou un ion issu d'une amine organique, et
▪ X⁻représente un contre ion anionique organique ou inorganique, tel que celui choisi parmi les halogénures, acétates, phosphates, nitrates, alkyl(C₁-C₄)sulfates, alkyl(C₁-C₄)- ou alkyl(C₁-C₄)aryl-sulfonates, en particulier méthylsulfate et éthylsulfate ; ou alors M⁺ et X⁻ sont absents ;

R_{a'}-C(O)-NH-CH₂-CH₂-N(B)(B') **(B2)**

Formule dans laquelle :
▪ B représente le groupe -CH₂-CH₂-O-X' ;
▪ B' représente le groupe -(CH₂)_{z}Y', avec z = 1 ou 2 ;
▪ X' représente le groupe -CH₂-C(O)OH, -CH₂-C(O)OZ', -CH₂-CH₂-C(O)OH, -CH₂-CH₂-C(O)OZ', ou un atome d'hydrogène ;
▪ Y' représente le groupe -C(O)OH, -C(O)OZ', -CH₂-CH(OH)-SO₃H ou le groupe -CH₂-CH(OH)-SO₃-Z' ;
▪ Z' représente un contre ion cationique issu d'un métal alcalin ou alcalinoterreux, tel que le sodium, un ion ammonium ou un ion issu d'une amine organique ;
▪ R_{a'} représente un groupe alkyle ou alcényle en C₁₀-C₃₀ d'un acide R_{a'}-C(O)OH de préférence présent dans l'huile de coprah ou dans l'huile de lin hydrolysée, un groupe alkyle, notamment en C₁₇ et sa forme iso, un groupe en C₁₇ insaturé.

Ces composés sont classés dans le dictionnaire CTFA, 5ème édition, 1993, sous les dénominations cocoamphodiacétate de disodium, lauroamphodiacétate de disodium, caprylamphodiacétate de disodium, capryloamphodiacétate de disodium, cocoamphodipropionate de disodium, lauroamphodipropionate de disodium, caprylamphodipropionate de disodium, capryloamphodipropionate de disodium, acide lauroamphodipropionique, acide cocoamphodipropionique.

A titre d'exemple, on peut citer le cocoamphodiacétate commercialisé par la société RHODIA sous la dénomination commerciale MIRANOL® C2M concentré. On peut aussi utiliser des composés de formule **(B'2)** ;

R_{a"}-NH-CH(Y")-(CH₂)n-C(O)-NH-(CH₂)n'-N(R_{d})(Rₑ) **(B'2)**

Formule dans laquelle :
▪ Y" représente le groupe -C(O)OH, -C(O)OZ", -CH₂-CH(OH)-SO₃H ou le groupe -CH₂-CH(OH)-SO₃-Z" ;
▪ R_{d} et Rₑ, indépendamment l'un de l'autre, représentent un radical alkyle ou hydroxyalkyle en C₁_C₄
▪ Z" représente un contre ion cationique issu d'un métal alcalin ou alcalinoterreux, tel que le sodium, un ion ammonium ou un ion issu d'une amine organique ;
▪ R_{a"} représente un groupe alkyle ou alcényle en C₁₀-C₃₀ d'un acide R_{a"}-C(O)OH de préférence présent dans l'huile de coprah ou dans l'huile de lin hydrolysée.
▪ n et n', indépendamment l'un de l'autre,désignet un nombre entier allant de 1 à 3.

Parmi les composés de formule **(B'2)** on peut citer le composé classé dans le dictionnaire CTFA sous la dénomination sodium diethylaminopropyl cocoaspartamide et commercialisé par la société CHIMEX sous l'appellation CHIMEXANE HB.

Parmi les tensioactifs amphotères ou zwittérioniques cités ci-dessus, on utilise de préférence les tensio-actifs de formule (2) seuls, ou en mélanges.

Conformément à un mode de réalisation particulier de l'invention, la teneur en tensioactif(s) amphotère(s) ou zwittérionique(s) varie de 0,05 à 30 % en poids, de préférence de 0,5 à 10 % en poids, et de manière plus préférée de 0,1 à 5 % en poids, par rapport au poids total de la composition.

La composition de l'invention peut en outre comprendre au moins un ingrédient cosmétique usuel, notamment choisi parmi les propulseurs; les huiles; les corps gras solides et notamment les esters en C₈-C₄₀, les acides en C₈-C₄₀; les alcools en C₈-C₄₀, les tensioactifs autres que ceux décrits précédemment, les filtres solaires; les agents hydratants; les agents antipelliculaires; les agents antioxydants; les agents chélatants; les agents nacrants et opacifiants; les agents plastifiants ou de coalescence; les charges; les silicones et en particulier les polydiméthylsiloxanes; les épaississants, polymériques ou non; les gélifiants; les émulsionnants; les polymères notamment conditionneurs ou coiffants; les parfums; les agents d'alcalinisation ou d'acidification;les silanes ;les agents de réticulation. La composition peut bien évidemment comprendre plusieurs ingrédients cosmétiques figurant dans la liste ci-dessus.

La composition peut notamment comprendre un ou plusieurs solvants organiques notamment hydrosolubles tels que les alcools en C₁-C₇; on peut notamment citer les monoalcools aliphatiques en C₁-C₇ ou aromatiques en C₆-C₇, les polyols et les éthers de polyols en C₃-C₇, qui peuvent être employés seuls ou en mélange avec de l'eau.

Selon leur nature et la destination de la composition, les ingrédients cosmétiques usuels peuvent être présents en des quantités usuelles, aisément déterminables par l'homme du métier, et qui peuvent être comprises, pour chaque ingrédient, entre 0,01 à 80% en poids. L'homme de métier veillera à choisir les ingrédients entrant dans la composition, ainsi que leurs quantités, de manière à ce qu'ils ne nuisent pas aux propriétés des compositions de la présente invention.

Le pH de la composition est inférieur à 4, et varie de préférence de 1 à 3, mieux de 1,7 à 3.

La composition selon l'invention se présente de préférence sous forme de gels de coiffage ou de soin, de lotions ou crèmes de soin, de conditionneurs, de masques, de sérums.

La composition selon l'invention peut être obtenue par mélange de plusieurs compositions.

Le procédé de l'invention comprend l'application de la composition décrite précédemment suivi d'une étape de lissage au fer des cheveux. Le lissage au fer est connu de l'état de la technique. Il consiste à lisser les cheveux avec une pince plate chauffante, généralement métallique. Les fers de lissage sont généralement utilisés à une température variant de 150 à 250°C.

Le procédé de l'invention peut comprendre d'autres étapes intermédiaires visant à améliorer le lissage des cheveux.

Selon un mode de réalisation particulier, le procédé de l'invention comprend l'application de la composition de l'invention sur cheveux secs, un temps de contact de la composition sur les cheveux compris entre 10 et 60 minutes, de préférences entre 20 et 40 minutes. Après ce temps de pose, on effectue un lissage à la brosse et au sèche-cheveux (brushing). On lisse ensuite les cheveux au fer à lisser à une température comprise entre 150 et 250°C, de préférence 210 à 230°C.

Le procédé de l'invention peut comprendre l'application d'autres agents capillaires en pré- ou post traitement à l'acide glyoxylique. Notamment, il peut comprendre l'application d'un soin de conditionnement en post traitement.

Selon un autre mode de réalisation, le procédé de lissage des cheveux comprend une étape de lavage des cheveux puis de séchage au sèche-cheveux avant application de la composition de l'invention. Selon ce mode de réalisation particulier, on retrouve ensuite les étapes décrites ci-dessus telles que le temps de contact de la composition, le brushing, le lissage au fer à lisser, l'application d'un agent de conditionnement et le rinçage, toutes ces étapes pouvant être mise en oeuvre indépendamment l'une de l'autre .Selon un mode de réalisation particulier, le lissage au fer à lisser s'effectue en plusieurs passages sur les cheveux, en général 8 à 10 passages.

### Exemple

Composition selon l'invention: (les teneurs sont indiqués en matière première brute (en gramme du produit commercial indiqués)

| | |
|---|---|
| HYDROXYPROPYL METHYLCELLULOSE(METHOCEL F4M de DOW CHEMICAL) | 1 |
| Acide glyoxylique (50% en solution aqueuse) | 16 |
| DISODIUM COCOAMPHODIACETATE ( N-COCOYL AMIDOETHYL, N-ETHOXYCARBOXYMETHYL GLYCINATE DE SODIUM) solution aqueuse à 30 %( MIRANOL C2M CONC NP de RHODIA) | 2 |
| HOMOPOLYMERE DE CHLORURE DE METHACRYLATE D'ETHYL TRI-METHYL AMMONIUM RETICULE, EN DISPERSION DANS UN MEL. D'ESTERS A 50 % (SALCARE SC 96 de BASF) | 2,5 |
| Acide lactique | 2,5 |
| Hydroxide de sodium (10% en solution aqueuse) | 7 |
| BISAMINO PEG/PPG-41-3 AMINOETHYL PG-PROPYL DIMETHICONE à 30 % MA (SILSOFT A 843 de MOMENTIVE PERFORMANCE MATERIALS) | 2 |
| WATER | QSP |

| | |
|---|---|
| *pH 2.2 ± 0.2 | |

La composition ci-dessus est appliquée sur une mèche de cheveux présentant un niveau de frisure important. Après 20 minutes de pose sur les cheveux, on réalise un brushing avec séchoir de la mèche puis on lisse au moyen d'un fer à lisser. Le dégagement de fumée lors de l'utilisation du fer est faible.

La mèche est ensuite lavée puis séchée au sèche-cheveux. On obtient ainsi une mèche de cheveu lisse et sans frisottis.

## Revendications

1. Composition cosmétique comprenant
• au moins un composé dicarbonylé de formule (I) suivante : 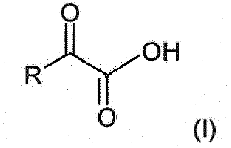 formule (I) dans laquelle
R représente un atome ou groupe choisi parmi i) hydrogène, ii) carboxy -C(O)OH, iii) alkyle linéaire ou ramifié en C₁-C₆ éventuellement substitué iv) phényle éventuellement substitué, v) benzyle éventuellement substitué, vi) un radical indolyl et vii) un radical imidazolylméthyle et ses tautomères,
et/ou ses dérivés choisis parmi les esters du ou des groupes carboxy, les amides du ou des groupes carboxy, les (thio)acétals et hémi(thio)acétals de la ou des fonctions carbonyles des composés de formule **(I)**, sous forme libre ou éventuellement sous forme de sels ou d'hydrates, et/ou ses hydrates et/ou les sels correspondants
• au moins un polymère cellulosique et
• au moins un tensioactif amphotère ou zwittérionique, la composition présentant un pH inférieur à 4.

2. Composition selon la revendication 1, dans laquelle le ou les composés dicarbonylés sont de formule **(I)** avec R représentant i) un atome d'hydrogène ou ii) un groupe alkyle linéaire ou ramifié en C₁-C₆ éventuellement substitué par un groupe carboxy.

3. Composition selon l'une quelconque des revendications précédentes dans laquelle le ou les composés dicarbonylés de formule **(I)** et/ou leurs dérivés et/ou leurs hydrates et/ou leurs sels sont choisis parmi l'acide glyoxylique, l'acide pyruvique, l'un de leurs dérivés, leurs sels et leurs hydrates, de préférence parmi l'acide glyoxylique, l'un de ses dérivés et les formes hydrate de ces composés.

4. Composition selon l'une quelconque des revendications précédentes, dans laquelle le ou les dérivés dicarbonylés de formule **(I)** et/ou leurs dérivés sont choisis parmi les esters d'acide glyoxylique, les amides d'acide glyoxylique, les (thio)acétals et hémi(thio)acétals d'acide glyoxylique, les (thio)acétals et hém(thio)acétals d'ester d'acide glyoxylique.

5. Composition selon l'une quelconque des revendications précédentes dans laquelle l'acide glyoxylique est sous forme d'hydrate.

6. Composition selon l'une quelconque des revendications précédentes, comprenant de 0,1 à 20% de composés dicarbonylés de formule (I) et/ou de ses dérivés en poids du poids total de la composition de préférence d'au moins 3 % en poids du poids total de la composition, de préférence de 3 à 10 % en poids du poids total de la composition.

7. Composition selon l'une quelconque des revendications précédentes, dans laquelle le polymère cellulosique est non ionique, de préférence choisi parmi les les (C₁-C₄)alkylcelluloses, les (poly)hydroxy(C₁-C₄)alkylcelluloses, les celluloses mixtes (poly)hydroxy(C₁-C₄)alkyl-(C₁-C₄)alkylcelluloses.

8. Composition selon l'une quelconque des revendications précédentes, dans laquelle le tensio-actif amphotère ou zwittérionique est choisi parmi les composés de formule **(B1)** suivante :
Rₐ-C(O)-NH-CH₂-CH₂-N⁺(R_{b})(R_{c})-CH₂C(O)O⁻, M⁺ , X⁻ **(B1)**
Formule dans laquelle :
▪ Rₐ représente un groupe alkyle ou alcényle en C₁₀-C₃₀ dérivé d'un acide RₐCOOH, de préférence présent dans l'huile de coprah hydrolysée, un groupe heptyle, nonyle ou undécyle ;
▪ R_{b} représente un groupe bêta-hydroxyéthyle ; et
▪ R_{c} représente un groupe carboxyméthyle ;
▪ M⁺ représente un contre ion cationique issu d'un métal alcalin, alcalinoterreux, tel que le sodium, un ion ammonium ou un ion issu d'une amine organique, et
▪ X⁻représente un contre ion anionique organique ou inorganique, tel que celui choisi parmi les halogénures, acétates, phosphates, nitrates, alkyl(C₁-C₄)sulfates, alkyl(C₁-C₄)- ou alkyl(C₁-C₄)aryl-sulfonates, en particulier méthylsulfate et éthylsulfate ; ou alors M⁺ et X⁻ sont absents .

9. Composition selon l'une quelconque des revendications 1 à 8, dans laquelle le tensio-actif amphotère ou zwittérionique est choisi parmi les composés de formule **(B2)** suivante
R_{a'}-C(O)-NH-CH₂-CH₂-N(B)(B') **(B2)**
Formule dans laquelle :
▪ B représente le groupe -CH₂-CH₂-O-X' ;
▪ B' représente le groupe -(CH₂)_{z}Y', avec z = 1 ou 2 ;
▪ X' représente le groupe -CH₂-C(O)OH, -CH₂-C(O)OZ', -CH₂-CH₂-C(O)OH, -CH₂-CH₂-C(O)OZ', ou un atome d'hydrogène ;
▪ Y' représente le groupe -C(O)OH, -C(O)OZ', -CH₂-CH(OH)-SO₃H ou le groupe -CH₂-CH(OH)-SO₃-Z' ;
▪ Z' représente un contre ion cationique issu d'un métal alcalin ou alcalinoterreux, tel que le sodium, un ion ammonium ou un ion issu d'une amine organique ;
R_{a'} représente un groupe alkyle ou alcényle en C₁₀-C₃₀ d'un acide R_{a'}-C(O)OH de préférence présent dans l'huile de coprah ou dans l'huile de lin hydrolysée, un groupe alkyle, notamment en C₁₇ et sa forme iso, un groupe en C₁₇ insaturé.

10. Composition selon l'une quelconque des revendications 1 à 8, dans laquelle le tensio-actif amphotère ou zwittérionique est choisi parmi les composés de formule **(B'2)** suivante;
R_{a"}-NH-CH(Y")-(CH₂)n-C(O)-NH-(CH₂)n'-N(R_{d})(Rₑ) **(B'2)**
Formule dans laquelle :
▪ Y" représente le groupe -C(O)OH, -C(O)OZ", -CH₂-CH(OH)-SO₃H ou le groupe -CH₂-CH(OH)-SO₃-Z" ;
▪ R_{d} et Rₑ, indépendamment l'un de l'autre, représentent un radical alkyle ou hydroxyalkyle en C₁_C₄
▪ Z" représente un contre ion cationique issu d'un métal alcalin ou alcalinoterreux, tel que le sodium, un ion ammonium ou un ion issu d'une amine organique ;
▪ R_{a"} représente un groupe alkyle ou alcényle en C₁₀-C₃₀ d'un acide R_{a"}-C(O)OH de préférence présent dans l'huile de coprah ou dans l'huile de lin hydrolysée.
▪ n et n', indépendamment l'un de l'autre, désignent un nombre entier allant de 1 à 3.

11. Composition selon l'une quelconque des revendications précédentes dans laquelle la teneur en tensioactif(s) amphotère(s) ou zwittérionique(s) varie de 0,05 à 30 % en poids, de préférence de 0,5 à 10 % en poids, et de manière plus préférée de 0,1 à 5 % en poids, par rapport au poids total de la composition.

12. Composition selon l'une quelconque des revendications précédentes **caractérisée en ce qu'**elle est aqueuse et comprend de l'eau à une concentration de préférence allant de 5 à 98 %, mieux de 5 à 50 %, encore mieux de 10 à 40 % en poids par rapport au poids total de la composition.

13. Procédé de lissage des fibres kératiniques telles que les cheveux qui comprend l'application sur les cheveux de la composition selon l'une quelconque des revendications précédentes un temps de contact compris entre 10 et 60 minutes et suivi d'une étape de lissage au moyen d'un fer de lissage à une température d'au moins 150 °C, de préférence entre 150 et 250°C:

14. Utilisation de la composition selon l'une quelconque des revendications 1 à 12 pour le lissage/défrisage des fibres kératiniques telles que les cheveux.

## Patentansprüche

1. Kosmetische Zusammensetzung, umfassend
• mindestens eine Dicarbonylverbindung der nachstehenden Formel (I): 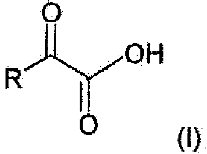 wobei in der Formel (I)
R für ein Atom bzw. eine Gruppe, das bzw. die aus i) Wasserstoff, ii) Carboxy -C(O)OH, iii) gegebenenfalls substituiertem linearem oder verzweigtem C₁-C₆-Alkyl, iv) gegebenenfalls substituiertem Phenyl, v) gegebenenfalls substituiertem Benzyl, vi) einem Indolylrest und vii) einen Imidazolylmethylrest und Tautomeren davon ausgewählt ist, steht, und/oder Derivate davon, die aus Estern der Carboxygruppe bzw. der Carboxygruppen, Amiden der Carboxygruppe bzw. der Carboxygruppen, (Thio)acetalen und Hemi(thio)acetalen der Carbonylfunktion bzw. der Carbonylfunktionen der Verbindungen der Formel (I) in freier Form oder gegebenenfalls in Form von Salzen oder Hydraten ausgewählt sind, und/oder Hydrate davon und/oder die entsprechenden Salze,
• mindestens ein Cellulosepolymer und
• mindestens ein amphoteres oder zwitterionisches Tensid,
wobei die Zusammensetzung einen pH-Wert von weniger als 4 aufweist.

2. Zusammensetzung nach Anspruch 1, wobei die Dicarbonylverbindung bzw. die Dicarbonylverbindungen die Formel (I) aufweist bzw. aufweisen, wobei R für i) ein Wasserstoffatom oder ii) eine gegebenenfalls durch eine Carboxygruppe substituierte lineare oder verzweigte C₁-C₆-Alkylgruppe steht.

3. Zusammensetzung nach einem der vorhergehenden Ansprüche, wobei die Dicarbonylverbindung bzw. die Dicarbonylverbindungen der Formel (I) und/oder Derivate davon und/oder Hydrate davon und/oder Salze davon aus Glyoxylsäure, Brenztraubensäure, einem Derivat davon, Salzen davon und Hydraten davon, vorzugsweise aus Glyoxylsäure, einem Derivat davon und den Hydratformen dieser Verbindungen, ausgewählt ist bzw. sind.

4. Zusammensetzung nach einem der vorhergehenden Ansprüche, wobei die Dicarbonylverbindung bzw. die Dicarbonylverbindungen der Formel (I) und/oder Derivate davon aus Glyoxylsäureestern, Glyoxylsäureamiden, Glyoxylsäure(thio)acetalen und -hemi(thio)acetalen und Glyoxylsäureester(thio)acetalen und -hemi(thio)-acetalen ausgewählt ist bzw. sind.

5. Zusammensetzung nach einem der vorhergehenden Ansprüche, wobei die Glyoxylsäure in Hydratform vorliegt.

6. Zusammensetzung nach einem der vorhergehenden Ansprüche, umfassend 0,1 bis 20 Gew.-% Dicarbonylverbindungen der Formel (I) und/oder Derivate davon, bezogen auf das Gesamtgewicht der Zusammensetzung, vorzugsweise mindestens 3 Gew.-%, bezogen auf das Gesamtgewicht der Zusammensetzung, vorzugsweise 3 bis 10 Gew.-%, bezogen auf das Gesamtgewicht der Zusammensetzung.

7. Zusammensetzung nach einem der vorhergehenden Ansprüche, wobei das Cellulosepolymer nichtionisch ist und vorzugsweise aus (C₁-C₄)Alkylcellulosen, (Poly)hydroxy(C₁-C₄)alkylcellulosen und gemischten (Poly)hydroxy(C₁-C₄)alkyl-(C₁-C₄)alkylcellulosen ausgewählt ist.

8. Zusammensetzung nach einem der vorhergehenden Ansprüche, wobei das amphotere oder zwitterionische Tensid aus den Verbindungen der folgenden Formel **(B1)** ausgewählt ist:
Rₐ-C(O)-NH-CH₂-CH₂-N⁺(R_{b})(R_{c})-CH₂C(O)O⁻, M⁺, X⁻ **(B1)**
wobei in der Formel:
▪ Rₐ für eine C₁₀-C₃₀-Alkyl- oder -Alkenylgruppe, die sich von einer Säure RₐCOOH, die vorzugsweise in hydrolysiertem Coprahöl vorliegt, ableitet, oder eine Heptyl-, Nonyl- öder Undecylgruppe steht;
▪ R_{b} für eine beta-Hydroxyethylgruppe steht; und
▪ R_{c} für eine Carboxymethylgruppe steht;
▪ M⁺ für ein kationisches Gegenion, das sich von einem Alkalimetall oder Erdalkalimetall, wie Natrium, ein Ammoniumion oder ein Ion, das sich von einem organischen Amin ableitet, steht und
▪ X⁻ für ein organisches oder anorganisches anionisches Gegenion steht, wie dasjenige, das aus Halogeniden, Acetaten, Phosphaten, Nitraten, (C₁-C₄)Alkylsulfaten, (C₁-C₄)Alkyl- oder (C₁-C₄)Alkylarylsulfonaten, insbesondere Methylsulfat und Ethylsulfat, ausgewählt ist; oder auch M⁺ und X⁻ fehlen.

9. Zusammensetzung nach einem der Ansprüche 1 bis 8, wobei das amphotere oder zwitterionische Tensid aus den Verbindungen der folgenden Formel **(B2)** ausgewählt ist:
R_{a'}-C(O)-NH-CH₂-CH₂-N(B)(B') **(B2)**
wobei in der Formel:
▪ B für die Gruppe -CH₂-CH₂-O-X' steht;
▪ B' für die Gruppe -(CH₂)_{z}Y' mit z = 1 oder 2 steht;
▪ X' für die Gruppe -CH₂-C(O)OH, -CH₂-C(O)OZ', -CH₂-CH₂-C(O)OH, -CH₂-CH₂-C(O)OZ' oder ein Wasserstoffatom steht;
▪ Y' für die Gruppe -C(O)OH, -C(O)OZ', -CH₂-CH(OH)-SO₃H oder die Gruppe -CH₂-CH(OH)-SO₃-Z' steht;
▪ Z' für ein kationisches Gegenion, das sich von einem Alkalimetall oder Erdalkalimetall, wie Natrium, ein Ammoniumion oder ein Ion, das sich von einem organischen Amin ableitet, steht;
R_{a'} für eine C₁₀-C₃₀-Alkyl- oder -Alkenylgruppe, die sich von einer Säure R_{a'}COOH, die vorzugsweise in Coprahöl oder in hydrolysiertem Leinöl vorliegt, ableitet, eine Alkylgruppe, insbesondere eine C₁₇-Alkylgruppe und deren Isoform oder eine ungesättigte C₁₇-Gruppe steht.

10. Zusammensetzung nach einem der Ansprüche 1 bis 8, wobei das amphotere oder zwitterionische Tensid aus den Verbindungen der folgenden Formel **(B'2)** ausgewählt ist:
R_{a"}-NH-CH(Y")-(CH₂)n-C(O)-NH-(CH₂)n'-N(R_{d})(Rₑ) **(B'2)**
wobei in der Formel:
▪ Y" für die Gruppe -C(O)OH, -C(O)OZ", -CH₂-CH(OH)-SO₃H oder die Gruppe -CH₂-CH(OH)-SO₃-Z" steht;
▪ R_{d} und Rₑ unabhängig voneinander für einen C₁-C₄-Alkyl- oder -Hydroxyalkylrest stehen;
▪ Z" für ein kationisches Gegenion, das sich von einem Alkalimetall oder Erdalkalimetall, wie Natrium, ein Ammoniumion oder ein Ion, das sich von einem organischen Amin ableitet, steht;
▪ R_{a"} für eine C₁₀-C₃₀-Alkyl- oder -Alkenylgruppe, die sich von einer Säure R_{a"}C(O)OH, die vorzugsweise in Coprahöl oder in hydrolysiertem Leinöl vorliegt, ableitet, steht;
▪ n und n' unabhängig voneinander für eine ganze Zahl im Bereich von 1 bis 3 stehen.

11. Zusammensetzung nach einem der vorhergehenden Ansprüche, wobei der Gehalt an amphoterem oder zwitterionischem Tensid bzw. amphoteren oder zwitterionischen Tensiden im Bereich von 0,05 bis 30 Gew.-%, vorzugsweise von 0,5 bis 10 Gew.-% und weiter bevorzugt 0,1 bis 5 Gew.-%, bezogen auf das Gesamtgewicht der Zusammensetzung, liegt.

12. Zusammensetzung nach einem der vorhergehenden Ansprüche, **dadurch gekennzeichnet, dass** sie wässrig ist und Wasser in einer Konzentration von vorzugsweise 5 bis 98 Gew.-%, besser 5 bis 50 Gew.-% und noch besser 10 bis 40 Gew.-%, bezogen auf das Gesamtgewicht der Zusammensetzung, umfasst.

13. Verfahren zum Glätten von Keratinfasern wie dem Haar, bei dem man auf das Haar die Zusammensetzung nach einem der vorhergehenden Ansprüche über eine Kontaktzeit zwischen 10 und 60 Minuten aufbringt und danach einen Glättungsschritt mit einem Glätteisen bei einer Temperatur von mindestens 150 °C, vorzugsweise zwischen 150 und 250 °C, durchführt.

14. Verwendung der Zusammensetzung nach einem der Ansprüche 1 bis 12 zur Glättung/Entkräuselung von Keratinfasern wie dem Haar.

## Claims

1. Cosmetic composition comprising
• at least one dicarbonyl compound of formula (I) below 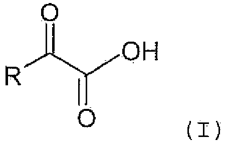 in which formula (I):
R represents an atom or group chosen from i) hydrogen, ii) carboxyl -C(O)OH, iii) optionally substituted linear or branched C₁-C₆ alkyl, iv) optionally substituted phenyl, v) optionally substituted benzyl, vi) an indolyl radical, and vii) an imidazolylmethyl radical, and tautomers thereof,
and/or derivatives thereof esters of the carboxy group(s), amides of the carboxy group(s) and (thio)acetals and hemi(thio)acetals of the carbonyl function(s) of the compounds of formula **(I),** in free form or optionally in the form of salts or of hydrates, and/or hydrates thereof and/or the corresponding salts:
• at least one cellulose-based polymer and
• at least one amphoteric or zwitterionic surfactant,
the composition having a pH of less than 4.

2. Composition according to Claim 1, in which the dicarbonyl compound(s) are of formula **(I)** with R representing i) a hydrogen atom or ii) a linear or branched C₁-C₆ alkyl group optionally substituted with a carboxyl group.

3. Composition according to either one of the preceding claims, in which the dicarbonyl compound(s) of formula **(I)** and/or derivatives thereof and/or hydrates thereof and/or salts thereof are chosen from glyoxylic acid and pyruvic acid, a derivative thereof, salts thereof and hydrates thereof, preferably from glyoxylic acid, a derivative thereof and the hydrate forms of these compounds.

4. Composition according to any one of the preceding claims, in which the dicarbonyl derivative(s) of formula **(I)** and/or derivatives thereof are chosen from glyoxylic acid esters, glyoxylic acid amides, glyoxylic acid (thio)acetals and hemi(thio)acetals, and glyoxylic acid ester (thio)acetals and hemi(thio)acetals.

5. Composition according to any one of the preceding claims, in which the glyoxylic acid is in hydrate form.

6. Composition according to any one of the preceding claims, comprising from 0.1% to 20% of dicarbonyl compounds of formula (I) and/or derivatives esters of the carboxy group(s), amides of the carboxy group(s) and (thio)acetals and hemi(thio)acetals of the carbonyl function(s) of the compounds of formula **(I),** in free form or optionally in the form of salts or of hydrates.

7. Composition according to any one of the preceding claims, in which the cellulose-based polymer is nonionic, preferably chosen from (C₁-C₄)alkylcelluloses, (poly)hydroxy(C₁-C₄)alkylcelluloses and mixed (poly)hydroxy(C₁-C₄)alkyl-(C₁-C₄)alkylcelluloses.

8. Composition according to any one of the preceding claims, in which the amphoteric or zwitterionic surfactant is chosen from the compounds of formula **(B1)** below:
Rₐ-C(O)-NH-CH₂-CH₂-N⁺(R_{b})(R_{c})-CH₂C(O)O⁻, M⁺, X⁻ **(B1)**
in which formula:
□ Rₐ represents a C₁₀-C₃₀ alkyl or alkenyl group derived from an acid Rₐ-COOH preferably present in hydrolyzed copra oil, or a heptyl, nonyl or undecyl group;
□ R_{b} represents a β-hydroxyethyl group; and
□ R_{c} represents a carboxymethyl group;
□ M⁺ represents a cationic counterion derived from an alkali metal or alkaline-earth metal, such as sodium, an ammonium ion or an ion derived from an organic amine, and
□ x⁻ represents an organic or mineral anionic counterion, such as that chosen from halides, acetates, phosphates, nitrates, (C₁-C₄)alkyl sulfates, (C₁-C₄)alkyl- or (C₁-C₄)alkylaryl sulfonates, in particular methyl sulfate and ethyl sulfate; or alternatively M⁺ and X⁻ are absent.

9. Composition according to any one of Claims 1 to 8, in which the amphoteric or zwitterionic surfactant is chosen from the compounds of formula **(B2)** below:
R_{a'}-C(O)-NH-CH₂-CH₂-N(B)(B') **(B2)**
in which formula:
▪ B represents the group -CH₂-CH₂-O-X' ;
▪ B¹ represents the group -(CH₂)_{z}Y', with z = 1 or 2;
▪ X' represents the group -CH₂-C(O)OH, -CH₂-C(O)OZ', -CH₂-CH₂-C(O)OH or
-CH₂-CH₂-C(O)OZ', or a hydrogen atom;
▪ Y' represents the group -C(O)OH, -C(O)OZ', -CH₂-CH(OH)-SO₃H or the group -CH₂-CH(OH)-SO₃-Z' ;
▪ Z' represents a cationic counterion derived from an alkali metal or alkaline-earth metal, such as sodium, an ammonium ion or an ion derived from an organic amine;
R_{a'} represents a C₁₀-C₃₀ alkyl or alkenyl group derived from an acid
R_{a'}-C(O)OH, which is preferably present in coconut oil or in hydrolyzed linseed oil, an alkyl group, especially a C₁₇ group and its iso form, or an unsaturated C₁₇ group.

10. Composition according to any one of Claims 1 to 8, in which the amphoteric or zwitterionic surfactant is chosen from the compounds of formula **(B'2)** below:
R_{a"}-NH-CH(Y")-(CH₂)n-C(O)-NH-(CH₂)n'-N(R_{d})(Rₑ) **(B'2)**
in which formula:
▪ Y" represents the group -C(O)OH, -C(O)OZ", -CH₂-CH(OH)-SO₃H or the group -CH₂-CH(OH)-SO₃-Z" ;
▪ R_{d} and Rₑ represent, independently of each other, a C₁-C₄ alkyl or hydroxyalkyl radical;
▪ Z" represents a cationic counterion derived from an alkali metal or alkaline-earth metal, such as sodium, an ammonium ion or an ion derived from an organic amine;
▪ R_{a"} represents a C₁₀-C₃₀ alkyl or alkenyl group derived from an acid
R_{a"}-C(O)OH, which is preferably present in coconut oil or in hydrolyzed linseed oil;
▪ n and n' denote, independently of each other, an integer ranging from 1 to 3.

11. Composition according to any one of the preceding claims, in which the content of amphoteric or zwitterionic surfactant(s) ranges from 0.05% to 30% by weight, preferably from 0.5% to 10% by weight and more preferably from 0.1% to 5% by weight, relative to the total weight of the composition.

12. Composition according to any one of the preceding claims, **characterized in that** it is aqueous and comprises water in a concentration preferably ranging from 5% to 98%, better still from 5% to 50% and even better still from 10% to 40% by weight relative to the total weight of the composition.

13. Process for straightening keratin fibers such as the hair, which comprises the application to the hair of the composition according to any one of the preceding claims, for a contact time of between 10 and 60 minutes, followed by a straightening step using a straightening iron at a temperature of at least 150°C, preferably between 150 and 250°C.

14. Use of the composition according to any one of Claims 1 to 12, for straightening/relaxing keratin fibers such as the hair.
